# FASCICULE DE BREVET EUROPEEN

(11) **EP 2 931 709 B1**
(45) Date de publication et mention de la délivrance du brevet: **02.10.2019**
(21) Numéro de dépôt: 13820801.2
(22) Date de dépôt: 11.12.2013
(51) Int. Cl.: C07D 233/56, C07D 233/58, C07D 233/60

(54) **PROCEDE DE PREPARATION DE SELS D'IMIDAZOLIUM DISSYMETRIQUES**
VERFAHREN ZUR HERSTELLUNG VON UNSYMMETRISCHEN IMIDAZOLIUMSALZEN
PROCESS FOR PREPARING ASYMMETRICAL IMIDAZOLIUM SALTS

(30) Priorité: 12.12.2012 FR 1261969
(43) Date de publication de la demande: 21.10.2015
(73) Titulaire: Ecole Nationale Supérieure de Chimie de Rennes, 35708 Rennes Cedex 7 (FR); Centre National de la Recherche Scientifique (C.N.R.S.), 75794 Paris Cedex 16 (FR)
(72) Inventeur: MAUDUIT, Marc, F-35500 Vitre (FR); BASLE, Olivier, F-35000 Rennes (FR); ROUEN, Mathieu, F-27430 Muids (FR)
(74) Mandataire: Cabinet Becker et Associés
(86) Numéro de dépôt international: PCT/FR2013/053036
(87) Numéro de publication internationale: WO 2014/091156

(56) Documents cités:
- WO-A2-02/094883
- WO-A2-2009/074535
- DIRK MEYER ET AL: "Palladium Complexes with Pyrimidine-Functionalized N-Heterocyclic Carbene Ligands: Synthesis, Structure and Catalytic Activity", ORGANOMETALLICS, vol. 28, no. 7, 13 avril 2009 (2009-04-13) , pages 2142-2149, XP055054921, ISSN: 0276-7333, DOI: 10.1021/om8009238
- ALOIS FÜRSTNER ET AL: "Convenient, scalable and flexible method for the preparation of imidazolium salts with previously inaccessible substitution patterns", CHEMICAL COMMUNICATIONS, no. 20, 1 janvier 2006 (2006-01-01), page 2176, XP055054925, ISSN: 1359-7345, DOI: 10.1039/b604236h
- REN ET AL: "A new class of o-hydroxyaryl-substituted N-heterocyclic carbene ligands and their complexes with palladium", JOURNAL OF ORGANOMETALLIC CHEMISTRY, ELSEVIER-SEQUOIA S.A. LAUSANNE, CH, vol. 692, no. 10, 29 mars 2007 (2007-03-29), pages 2092-2098, XP022008542, ISSN: 0022-328X, DOI: 10.1016/J.JORGANCHEM.2007.01.028
- ROBERTO FERNANDO DE SOUZA ET AL: "Alternative Synthesis of a Dialkylimidazolium Tetrafluoroborate Ionic Liquid Mixture and its Use in Poly(acrylonitrile-butadiene) Hydrogenation", ADVANCED SYNTHESIS & CATALYSIS, WILEY VCH VERLAG, WEINHEIM, DE, vol. 344, no. 2, 1 février 2002 (2002-02-01), pages 153-155, XP008111211, ISSN: 1615-4150, DOI: _

## Description

La présente invention vise des sels d'imidazolium dissymétriques et leur procédé de préparation.

Les diaminocarbènes N-hétérocycliques ou NHC (en anglais *N-Heterocyclic Carbene*) sont des ligands vastement utilisés en catalyse organométallique. Ceci est dû au fait que les ligands diaminocarbènes N-hétérocycliques peuvent former des espèces hautement réactives lorsqu'ils sont associés à un métal. Ces espèces sont alors appelées des complexes métalliques diaminocarbèniques.

Parmi les nombreux ligands, on peut notamment citer les ligands de type imidazolidine-2-ylidene (NHC saturé), et les ligands de type imidazoline-2-ylidene (NHC insaturé). En effet, ces ligands forment ensemble avec un métal des complexes particulièrement adaptés pour la catalyse organométallique. Les publications NHCs in Synthesis, S. P. Nolan, Ed., 2006, Wiley-VCH; NHCs in Transition Métal Catalysis, F. Glorius, Ed. 2006, Springer ; N-Heterocyclic Carbenes, S. D. Diez-Gonzalez, Ed. 2011, RSC Catalysis Series, RSC Publishing, divulguent des complexes métalliques diaminocarbèniques de ce type.

Il est aujourd'hui démontré que les complexes métalliques diaminocarbèniques ont permis, non-seulement d'augmenter le rendement de certaines réactions chimiques, mais aussi de réaliser des réactions chimiques jusqu'alors inexistantes. Les complexes métalliques diaminocarbèniques ont par exemple permis d'améliorer les rendements de la majorité des réactions métallo-catalysées, et notamment dans des réactions de couplages C-C, C-N, C-O, C-S etc... Ces réactions de couplage sont largement utilisées dans les procédés industriels de chimie fine comme le décrit la publication Dunetz et al., Chem. Rev. 2011, 111, 2177-2250.

Toutefois, c'est certainement en métathèse des oléfines que les métaux portant des ligands diaminocarbènes N-hétérocycliques ont le plus apporté en termes d'amélioration dans les rendements de réaction, tout en permettant de réduire significativement la charge catalytique nécessaire et suffisante pour catalyser la réaction. Ceci est notamment rapporté dans Grela et al., Chem. Rev. 2009, 109, 3708-3742.

De manière générale, l'état de la technique montre que ce sont des ligands diaminocarbéniques imidazolidine-2-ylidene ou imidazoline-2-ylidene 1,3-disubstitués symétriques qui sont impliqués dans les systèmes catalytiques organométalliques (cf. publications citées ci-dessus).

Néanmoins, certaines divulgations montrent que des ligands diaminocarbéniques imidazolidine-2-ylidene ou imidazoline-2-ylidene 1,3-disubstitués dissymétriques, donc portant des groupements carbonés non-identiques, peuvent également être impliqués dans les systèmes catalytiques organométalliques. Ainsi, la publication Blechert et al., Dalton Trans. 2012, 41, 8215-8225 montre de bonnes réactivités et sélectivités pour des ligands carbéniques dissymétriques. La publication Grubbs et al., J. Am. Chem. Soc. 2011, 113, 7490-7496 montre par ailleurs que de bonnes réactivités et sélectivités peuvent être observées lorsqu'un ligand diaminocarbène N-hétérocyclique porte un substituant aromatique d'une part et un groupement alkyle d'autre part.

La nature et le choix des groupements carbonés substituants restent toutefois très limités en ce qui concerne les ligands diaminocarbéniques imidazoline-2-ylidene 1,3-disubstitués dissymétriques. Ceci est dû en particulier à la difficulté de la synthèse des sels d'imidazolium dissymétriques précurseurs.

En effet, pour synthétiser un ligand diaminocarbénique imidazoline-2-ylidene 1,3-disubstitué dissymétrique il est crucial de générer d'abord un sel d'imidazolium précurseur. Cette synthèse est complexe et demande, soit un nombre très élevé d'opérations chimiques (4 à 6 opérations chimiques distinctes), soit un choix préalable limité des groupements carbonés substituants.

La publication Organ et al., Angew. Chem. Int. Ed., 2007, 46, 2768-2813., et la publication César et al. Chem. Rev. 2011, 111, 2701-2733 montrent la complexité et les limites de la synthèse de ligands diaminocarbéniques imidazoline-2-ylidene 1,3-disubstitués dissymétriques.

Les ligands diaminocarbéniques imidazoline-2-ylidene 1,3-disubstitués dissymétriques présentent un grand intérêt scientifique et économique, mais ne peuvent pas à ce jour être synthétisés de manière compétitive au plan industriel car le nombre d'opérations chimiques est bien trop élevé notamment. De plus, le choix des groupements substituants non-identiques est limité.

La présente invention vient améliorer la situation.

Ainsi les Demanderesses ont mis au point un procédé de préparation d'un sel d'imidazolium dissymétrique intermédiaire de formule 1A : 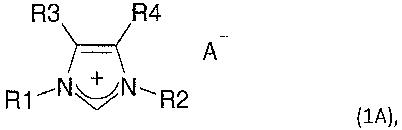 dans laquelle R1 est un groupement aromatique, R2 est choisi parmi un groupement alkyle aliphatique secondaire cyclique et un groupement hétéroalkyle, R3 et R4 sont choisis indépendamment l'un de l'autre dans le groupe constitué d'hydrogène, d'halogénure et d'un groupement alkyle, et A⁻ est un anion. Le procédé de l'invention est réalisé en une seule opération et comprend les étapes suivantes :
a. former un mélange réactionnel par la mise en contact d'un équivalent (1 eq) d'une aniline de formule 2 : 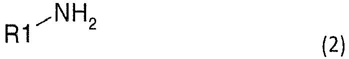 avec un équivalent (1 eq) d'un composé de formule 3 : 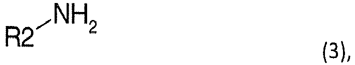 en présence d'au moins quatre virgule cinq équivalents (4,5 eq) d'un acide de Brønsted de formule 4 :

   AH (4);
b. former une solution comprenant un équivalent (1 eq) d'un dicarbonyle de formule 5 : 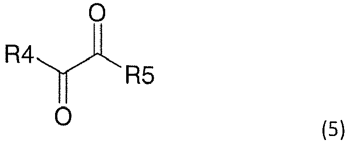 un équivalent (1 eq) de formaldéhyde, et au moins quatre virgule cinq équivalents (4,5 eq) de l'acide de Brønsted de formule 4, porter ladite solution à environ 80°C et y ajouter le mélange réactionnel formé à l'étape a. ;
c. laisser sous agitation pendant au moins 2 heures à environ 80°C ; et
d. isoler le sel d'imidazolium dissymétrique intermédiaire de formule 1A.

Le procédé de l'invention synthétise en une seule opération chimique un sel d'imidazolium dissymétrique intermédiaire qui permet notamment de former des sels d'imidazolium 1,3-disubstitués portant un groupement aromatique d'une part, et un groupement cycloalkyle ou hétéroalkyle d'autre part.

Ainsi, l'invention vise également un procédé de préparation d'un sel d'imidazolium dissymétrique de formule 1B : 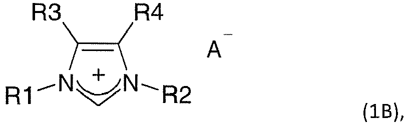 dans laquelle R1 est un groupement aromatique, R2 est choisi parmi un groupement alkyle aliphatique secondaire cyclique et un groupement hétéroalkyle, R3 et R4 sont choisis indépendamment l'un de l'autre dans le groupe constitué d'hydrogène, d'halogénure et d'un groupement alkyle, et A⁻ est choisi parmi le groupe constitué d'un anion tetrafluoroborate, un anion hexafluorophosphate, un anion hexafluoroantimoine, un anion tetrakis[(3,5-trifluorométhyl)phényl]borate et un anion halogénure, ledit procédé comprenant les étapes a. à d. citées ci-dessus, et comprenant en outre les étapes de :
e. ajouter un équivalent (1eq) d'un sel inorganique et du solvant organique, préférentiellement du dichlorométhane, au sel d'imidazolium dissymétrique intermédiaire isolé à l'étape d. ;
f. laisser sous agitation à température ambiante pendant au moins une heure et réaliser une extraction eau/solvant organique suivi d'une évaporation dudit solvant organique ;
g. précipiter par un solvant organique polaire, puis isoler le sel d'imidazolium dissymétrique de formule 1B.

Selon un mode de réalisation, le sel inorganique de l'étape e. du procédé de préparation pour le sel d'imidazolium dissymétrique de formule 1B est choisi parmi le groupe constitué de tetrafluoroborate de potassium, de tetrafluoroborate de sodium, de tetrafluoroborate de lithium, de tetrafluoroborate d'hydrogène, de tetrafluoroborate d'ammonium, de hexafluorophosphate de potassium, de hexafluorophosphate de sodium, de hexafluorophosphate de lithium, de hexafluorophosphate d'hydrogène, de hexafluorophosphate d'ammonium, d'hexafluoroantimoine d'argent, d'hexafluoroantimoine de potassium, d'hexafluoroantimoine de sodium, d'hexafluoroantimoine de lithium, de tetrakis[(3,5-trifluorométhyl)phényl]borate de potassium, de tetrakis[(3,5-trifluorométhyl)phényl]borate de sodium et de tetrakis[(3,5-trifluorométhyl)phényl]borate de lithium et d'acide halogénique.

Le sel d'imidazolium dissymétrique intermédiaire préparé selon le procédé de l'invention permet également de former des sels zwitterionique d'imidazolium 1,3-disubstitués portant un groupement aromatique d'une part, et un groupement hétéroalkyl d'autre part.

Ainsi, l'invention vise également un procédé de préparation d'un sel d'imidazolium dissymétrique de formule 1C : 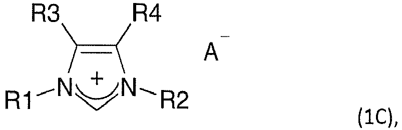 dans laquelle R1 est un groupement aromatique, R2 est un groupement hétéroalkyle, R3 et R4 sont choisis indépendamment l'un de l'autre dans le groupe constitué d'hydrogène, d'halogénure et d'un groupement alkyle, et A⁻ est une charge négative sur le groupement R2, ledit procédé comprenant les étapes a. à d. citées ci-dessus, et comprenant en outre les étapes de :
h. ajouter au moins dix équivalents (10 eq) d'une base carbonatée au sel d'imidazolium dissymétrique intermédiaire isolé à l'étape d. ;
i. isoler le sel d'imidazolium dissymétrique de formule 1C.

Selon un mode de réalisation, la base carbonatée de l'étape h. du procédé de préparation du sel d'imidazolium dissymétrique de formule 1C est choisie dans le groupe constitué de bicarbonate de sodium, de bicarbonate de potassium, d'hydrogénocarbonate de sodium et d'hydrogénocarbonate de potassium.

De manière générale, à l'étape h. il faut ajouter au moins dix équivalents (10 eq) d'une base carbonatée. Plus précisément il faut prévoir l'ajout d'un équivalent supplémentaire comparé aux équivalents d'acide de Brønsted ajouté aux étapes a. et b. ; en d'autres termes, si l'on avait ajouté 5 équivalents d'acide de Brønsted à l'étape a. et 5 équivalents d'acide de Brønsted à l'étape b. (à savoir 10 équivalents en totalité), il faut prévoir l'ajout de 11 équivalents d'une base carbonatée pour former le zwitterion.

Selon un mode de réalisation, l'acide de Brønsted de formule 4 des étapes a. et b. est l'acide acétique. Ceci permet un bon rendement pour l'obtention du sel intermédiaire.

Avantageusement, dans lesdites formules 1A et 1B, R1 est choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle ; et R2 est choisi dans le groupe constitué de cyclopentyle, de cyclohexyle, de cyclooctyle, de cyclodecyle, de cyclododecyle, et de cyclopentadecyle. En effet, un choix parmi ces groupements permet une bonne stabilité stérique.

Avantageusement, dans ladite formule 1C, R1 est choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle ; et R2 est choisi dans le groupe constitué de 3-methylbutanoate et 3,3-dimethylbutanoate. R2 peut, par ailleurs, être un hétéroalkyle cyclique tel qu'un polyéther cyclique par exemple.

Selon un mode de réalisation, R3 et R4 sont chacun l'hydrogène.

La présente invention décrit également une nouvelle catégorie de sel d'imidazolium dissymétrique. Ces nouveaux sels peuvent être obtenus par le procédé selon l'invention. Ainsi, l'invention vise un sel d'imidazolium dissymétrique de formule 1D 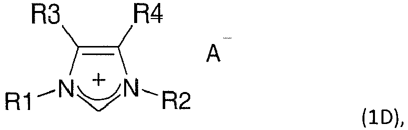 dans lequel R1 est un groupement aromatique choisi dans le groupe constitué de 2,4,6-triméthylphényle, de 3,5-dinitrophényle, de 2,4,6-tris(trifluorométhyle)phényle, de 2,4,6-trichlorophényle et de hexafluorophényle, R2 est un groupement alkyle aliphatique secondaire cyclique, R3 et R4 sont choisis indépendamment l'un de l'autre dans le groupe constitué d'hydrogène, d'halogénure et d'un groupement alkyle, et A⁻ est un anion choisi parmi le groupe constitué d'un anion tetrafluoroborate, d'un anion hexafluorophosphate, d'un anion acétate, d'un anion hexafluoroantimoine, d'un anion tetrakis[(3,5-trifluorométhyl)phényl]borate et d'un anion halogénure. Préférentiellement, R1 est choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle, et R2 est choisi dans le groupe constitué de cyclopentyle, de cyclohexyle, de cycloheptyle, de cyclooctyle, de cyclodecyle, de cyclododecyle, et de cyclopentadecyle. Selon un mode de réalisation, R3 et R4 sont chacun l'hydrogène.

Dans un mode de réalisation préférentiel, A⁻ est un anion tetrafluoroborate, un anion hexafluorophosphate, un anion acétate, un anion hexafluoroantimoine, un anion tetrakis[(3,5-trifluorométhyl)phényl]borate ou un anion halogénure. Ceci permet une bonne réactivité et stabilité.

La présente invention fait réagir une aniline (amine aromatique) et une amine aliphatique (y compris les hétéroalkyles comportant une fonction amine) dans des conditions particulières et en présence de dicarbonyle, de formaldéhyde et d'acide de Brønsted.

La réaction générale de synthèse pour la préparation du sel d'imidazolium dissymétrique intermédiaire selon l'invention est représentée ci-dessous :

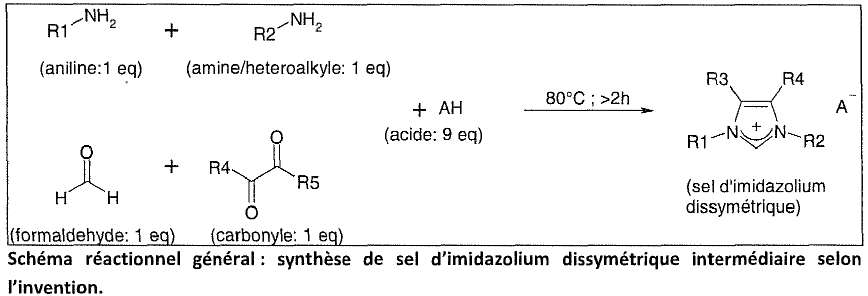

Il faut noter que lorsqu'on fait réagir un hétéroalkyle avec l'aniline, le formaldéhyde et le carbonyle, cet hétéroalkyle doit nécéssairement contenir une fonction amine. Ceci est notamment le cas des acides alpha-aminés ou des béta-amino-alcools.

L'opération unique de la synthèse chimique selon la présente invention comprend des sous-étapes. Ainsi, dans une première étape il est formé un mélange réactionnel par la mise en contact d'un équivalent (1 eq) d'une aniline avec un équivalent (1 eq) d'une amine (ou hétéroalkyle) en présence d'au moins quatre virgule cinq équivalents (4,5 eq) d'un acide de Brønsted. Ensuite, dans une seconde sous-étape, il est formé une solution comprenant un équivalent (1 eq) d'un dicarbonyle et un équivalent (1 eq) de formaldéhyde en présence d'au moins quatre virgule cinq équivalents (4,5 eq) d'un acide de Brønsted similaire à l'acide de Brønsted de la première sous-étape. Cette solution est alors portée à environ 80°C. Le mélange formé à la première sous-étape est ensuite lentement ajouté (par exemple en goutte à goutte) à la solution formé à la seconde sous-étape. Le mélange réactionnel obtenu est ensuite laissé sous agitation pendant au moins 2 heures à environ 80°C, avant d'isoler le sel d'imidazolium dissymétrique par extraction/filtration.

L'acide de Brønsted utilisé pour lesdites première et seconde sous-étapes est de préférence l'acide acétique pour des raisons de rendements. Dans ces conditions, on obtient un sel d'imidazolium dissymétrique composé d'un cation imidazolium et d'un anion acétate. Toutefois, d'autres acides forts peuvent être utilisés.

Les Demanderesses ont découvert non sans surprise que lorsqu'une amine aromatique et une amine aliphatique sont mises en contact dans les conditions décrites ci-dessus, leur différence de réactivité minimise fortement la formation des sels d'imidazolium 1,3-bis-aryliques et 1,3-bis-alkyles qui sont des sous-produits de la réaction résultant d'une réaction d'auto-condensation des amines présentes dans le milieu réactionnel.

Ainsi selon l'invention, la sélectivité peut atteindre un ratio de 1/30/1 en faveur du sel d'imidazolium dissymétrique souhaité (plus précisément : 1 partie du premier sel symétrique, 1 partie du deuxième sel d'imidazolium symétrique, et 30 parties du sel d'imidazolium dissymétrique). On pourra appeler ce sel un sel d'imidazolium dissymétrique intermédiaire dans la présente description. En effet, le sel d'imidazolium dissymétrique intermédiaire peut être soumis à des étapes supplémentaires afin de procéder à des étapes de conditionnement et le transformer partiellement le cas échéant.

En suivant ce procédé de synthèse mettant en contact une aniline et une amine aliphatique (y compris des hétéroalkyles), des sels précurseurs de diaminocarbènes de type 1-aryl-3-cycloalkyl-imidazoline-2-ylidene ou 1-aryl-3-hétéroalkyl-imidazoline-2-ylidene ont pu être isolés avec de bons rendements permettant une exploitation à l'échelle industrielle. De plus, la purification est réalisable par une cristallisation du sel d'imidazolium formé, ce qui augmente encore l'intérêt d'une exploitation à l'échelle industrielle.

Dans un premier mode de réalisation, le sel d'imidazolium dissymétrique intermédiaire obtenu par le procédé ci-dessus peut dans la suite être soumis à d'autres sous-étapes afin d'échanger l'anion issu desdites première et seconde sous-étapes contre un autre anion favorisant la réactivité ou la stabilité dans le temps. Parmi ces anions, on peut notamment citer l'anion tetrafluoroborate (BF₄⁻), l'anion hexafluorophosphate (PF₆⁻), l'anion hexafluoroantimoine (SbF₆⁻), l'anion tetrakis[(3,5-trifluorométhyl)phényl]borate (B(C₆F₅)₄)⁻), et les anions halogénures (X⁻, où X est un halogène). Cet échange d'ion est avantageusement réalisé lorsqu'on vise la synthèse de composés de type 1-aryl-3-cycloalkyl-imidazoline-2-ylidene. Ainsi, une troisième sous-étape peut consister en l'ajout d'un équivalent (1eq) d'un sel inorganique et du solvant (préférentiellement du dichlorométhane), au sel d'imidazolium dissymétrique intermédiaire isolé (après l'agitation pendant au moins 2 heures à environ 80°C et l'extraction/filtration décrite ci-dessus). Le mélange obtenu est laissé sous agitation à température ambiante jusqu'à évaporation dudit solvant. Ensuite, un solvant organique polaire permet de précipiter le sel pour l'isoler.

Dans un deuxième mode de réalisation, le sel d'imidazolium dissymétrique intermédiaire obtenu par le procédé décrit plus-haut peut être soumis à un traitement par une base carbonatée. Ce traitement pourra conduire à l'obtention du sel zwitterionique. Il faut noter que le sel sous forme de zwitterion est avantageusement obtenu lorsqu'on recherche la synthèse de composés de type 1-aryl-3-hétéroalkyl-imidazoline-2-ylidene. Ainsi une quatrième sous-étape peut consister en l'ajout d'au moins dix équivalents (10 eq) d'une base carbonatée au sel d'imidazolium dissymétrique intermédiaire isolé (après l'agitation pendant au moins 2 heures à environ 80°C et l'extraction/filtration décrite ci-dessus). Le sel zwitterionique d'imidazolium dissymétrique peut ensuite être isolé.

Le choix des groupements substituants est réalisé lors de la formation du sel d'imidazolium dissymétrique intermédiaire, à savoir dans l'opération principale comprenant lesdites première et deuxième sous-étapes.

Dans un mode de réalisation, R1 est de préférence choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle ; et R2 est de préférence choisi dans le groupe constitué de cyclopentyle, de cyclohexyle, de cycloheptyle, de cyclooctyle, de cyclodecyle, de cyclododecyle, et de cyclopentadecyle. Ces groupements offrent une bonne stabilité stérique. R3 et R4 sont de préférence chacun l'hydrogène.

En prenant comme exemple un groupement R1 de 2,4,6-triméthylphényle, un groupement R2 de cyclohexyle et des groupements R3 et R4 d'hydrogène, et en suivant le procédé décrit ci-dessus, on obtient un 1-aryle-3-cycloalkyle-imidazoline-2-ylidene de formule 6 ci-dessous :

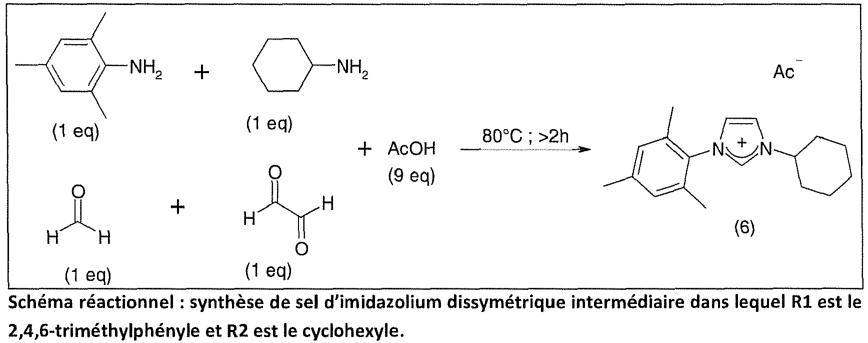

Pour déterminer la sélectivité de la réaction, le milieu réactionnel comprenant le sel 1-aryle-3-cycloalkyle-imidazoline-2-ylidene de formule 6 est refroidi à température ambiante. Ensuite de l'eau est additionnée suivis de solvant (par exemple dans de l'acétate d'éthyle ou du dichlorométhane). La phase aqueuse est extraite (par exemple trois fois) avec le solvant. Les phases organiques sont combinées et séchées sur un sel anionique (par exemple sur du sulfate de magnésium). Ensuite la phase organique est concentrée sous vide. Une analyse par résonnance magnétique nucléaire (RMN) du brut réactionnel peut permettre de déterminer la sélectivité de la réaction.

L'échange de l'anion acétate contre un autre anion peut être réalisé comme suit : le brut réactionnel est dissous dans du solvant (par exemple du dichlorométhane). On ajoute ensuite 1 équivalent du sel inorganique comprenant le contre anion souhaité (par exemple du tetrafluoroborate de potassium pour obtenir un contre anion tetrafluoroborate). Le mélange est ensuite agité à température ambiante pendant quelques heures (par exemple 3h). Ensuite, on réalise une extraction liquide/liquide, à savoir une extraction eau/solvant organique. Pour cela de l'eau est ajoutée, et la phase organique est séparée de la phase aqueuse. La phase aqueuse est lavée (par exemple trois fois) avec du solvant. Chaque phase organique est séchée sur un sel anionique (par exemple sur du sulfate de magnésium), et concentrée sous vide. On obtient généralement une huile marron, à laquelle on ajoute un solvant organique pour une précipitation (par exemple de l'acétate d'éthyle). Ensuite le mélange peut-être soumis à un traitement aux ultrasons pendant quelques minutes (par exemple 5 minutes). Un solide se forme lequel est filtré sur fritté, puis lavé avec du solvant (par exemple avec de l'acétate d'éthyle) pour obtenir l'imidazolium dissymétrique souhaité avec un anion souhaité (par exemple un anion tetrafluoroborate).

De manière générale, la précipitation ci-dessus par un solvant organique polaire peut se faire en utilisant l'acétate d'éthyle, le diéthylether, ou une cristallisation dans l'éthanol. De préférence, on utilise la précipitation par l'acétate d'éthyle.

Par le protocole opératoire ci-dessus, lequel ne comporte qu'une seule opération chimique, les Demanderesses ont pu synthétiser un grand nombre de nouvelles molécules. Notamment, les Demanderesses ont synthétisé les sels d'imidazolium dissymétrique de formule 7, 8, 9, 10, 11, 12, 13, 14 et 15 :

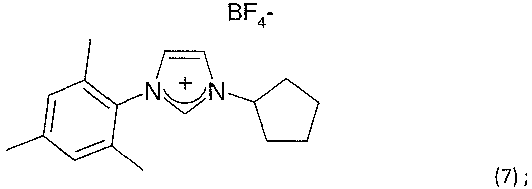

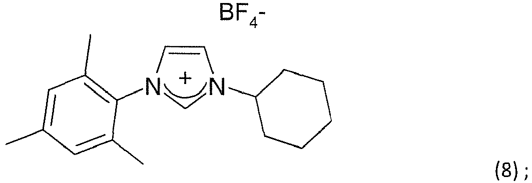

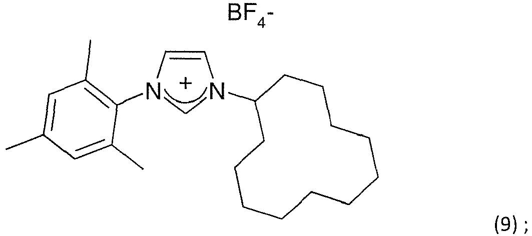

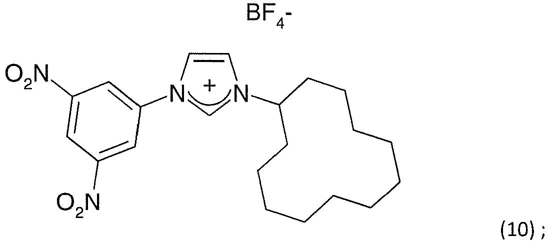

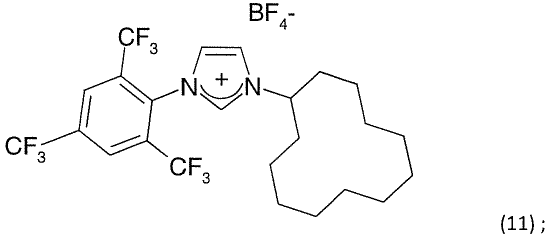

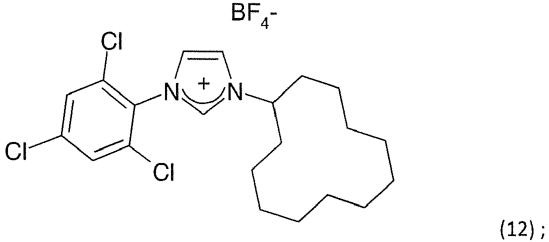

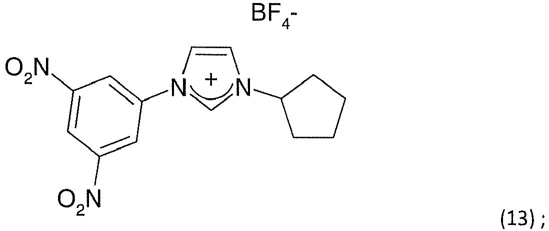

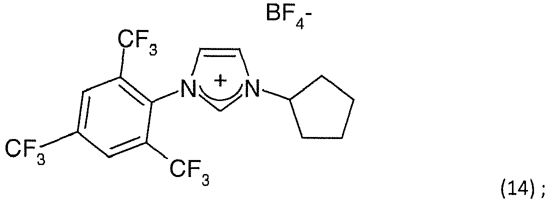

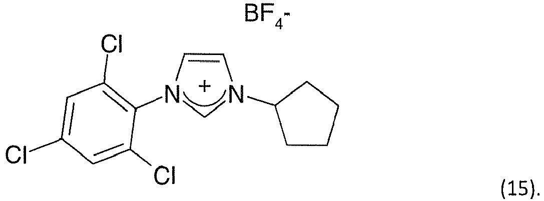

De manière générale les Demanderesses ont synthétisé des sels d'imidazolium dissymétriques de formule 1D 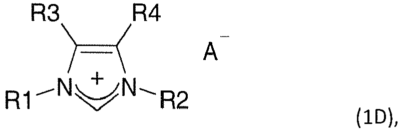
dans lequel R1 est un groupement aromatique, R2 est un groupement alkyle aliphatique secondaire cyclique, R3 et R4 sont choisis indépendamment l'un de l'autre dans le groupe constitué d'hydrogène, d'halogénure et d'un groupement alkyle, et A⁻ est un anion.
R1 peut notamment être le 2,4,6-triméthylphényle, le 3,5-dinitrophényle, le 2,4,6-tris(trifluorométhyle)phényle, le 2,4,6-trichlorophényle, ou le hexafluorophényle.
R2 peut notamment être le cyclopentyle, le cyclohexyle, le cycloheptyle, le cyclooctyle, le cyclodecyle, le cyclododecyle, ou le cyclopentadecyle.
R3 et R4 peuvent être chacun l'hydrogène. R3 et R4 peuvent aussi être des halogénures (par exemple un atome de chlore ou des halogénures d'alkyle). R3 et R4 peuvent aussi être des groupements alkyles (par exemple méthyle, éthyle propyle ou isopropyle).
L'anion A⁻ peut notamment être un anion tetrafluoroborate, un anion hexafluorophosphate, un anion acétate, un anion hexafluoroantimoine, un anion tetrakis[(3,5-trifluorométhyl)phényl]borate et un anion halogénure.

Par ailleurs, en prenant comme exemple un groupement R1 de 2,4,6-triméthylphényle, un groupement R2 hétéroalkyle (le composé de formule 3 étant la valine) et des groupements R3 et R4 d'hydrogène, et en suivant le procédé décrit plus haut, on obtient un 1-aryle-3-heteroalkyle-imidazoline-2-ylidene de formule 16 ci-dessous :

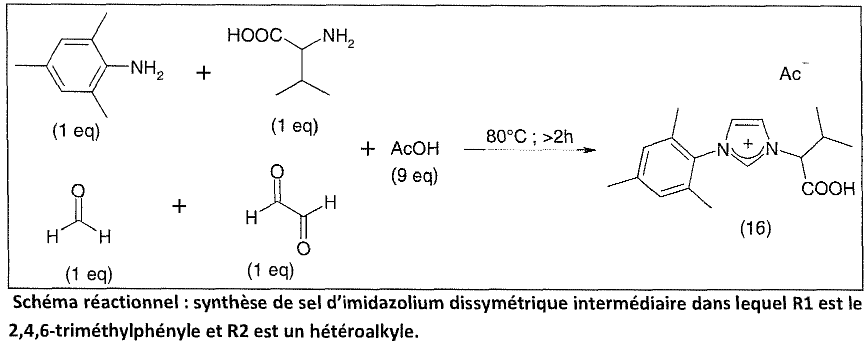

Pour déterminer la sélectivité de la réaction, le milieu réactionnel comprenant le sel 1-aryle-3-cycloalkyle-imidazoline-2-ylidene de formule 6 est refroidi à température ambiante. Ensuite de l'eau est additionnée suivis de solvant (par exemple dans de l'acétate d'éthyle ou du dichlorométhane). La phase aqueuse est extraite (par exemple en trois fois) avec le solvant. Les phases organiques sont combinées et séchées sur un sel anionique (par exemple sur du sulfate de magnésium). Ensuite la phase organique est concentrée sous vide. Une analyse par résonnance magnétique nucléaire (RMN) du brut réactionnel peut permettre de déterminer la sélectivité de la réaction.

L'échange de l'anion acétate contre un autre anion peut être réalisé comme suit : le brut réactionnel est dissous dans du solvant (par exemple du dichlorométhane). On ajoute ensuite 1 équivalent du sel inorganique comprenant le contre anion souhaité (par exemple du tetrafluoroborate de potassium pour obtenir un contre anion tetrafluoroborate). Le mélange est ensuite agité à température ambiante pendant quelques heures (par exemple 3h). Ensuite, de l'eau est ajoutée et la phase organique est séparée de la phase aqueuse. La phase aqueuse est lavée (par exemple trois fois) avec du solvant. Chaque phase organique est séchée sur un sel anionique (par exemple sur du sulfate de magnésium), et concentrée sous vide. On obtient généralement une huile marron, à laquelle on ajoute un solvant (par exemple de l'acétate d'éthyle). Ensuite le mélange peut-être soumis à un traitement aux ultrasons pendant quelques minutes (par exemple 5 minutes). Un solide se forme lequel est filtré sur fritté, puis lavé avec du solvant (par exemple avec de l'acétate d'éthyle) pour obtenir l'imidazolium dissymétrique souhaité avec un anion souhaité (par exemple un anion tetrafluoroborate).

Donc, en suivant le schéma réactionnel général selon l'invention, tout en utilisant des acides alpha-aminés ou des béta-amino-alcools (à savoir des hétéroalkyles en tant que composé de formule 3) qui sont de préférence énantiomèriquement purs, et en procédant à l'échange de contre-anion tel que décrit ci-dessus, les Demanderesses ont pu synthétiser un grand nombre de nouvelles molécules. Notamment, les Demanderesses ont synthétisé les sels d'imidazolium dissymétrique de formule 17 et 18 : 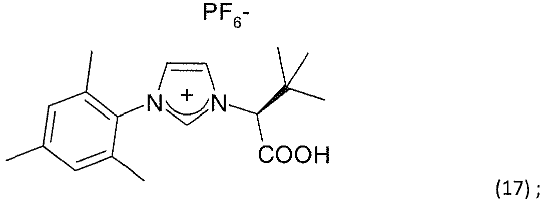 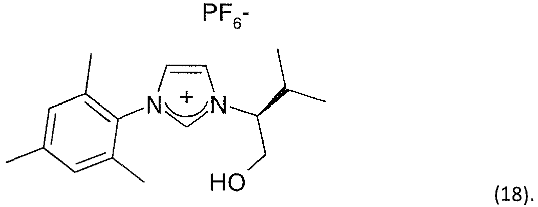 R2
D'autre part, lorsqu'on souhaite obtenir des sels sous forme de zwitterions il faut traiter le sel d'imidazolium dissymétrique intermédiaire, obtenu selon le schéma réactionnel général décrit plus haut, d'une manière particulière. Il faut prévoir une dissolution du sel d'imidazolium dissymétrique intermédiaire dans du solvant (par exemple dans de l'acétate d'éthyle). La phase organique est ensuite lavée (par exemple deux fois) avec une solution saturée de base carbonatée (par exemple avec une solution de bicarbonate de sodium). Les phases aqueuses sont combinées puis évaporées conduisant à un solide (généralement de couleur jaune). Le solide est ensuite repris dans du solvant (par exemple de l'acétone), puis filtré sur fritté (notamment pour éliminer les sels inorganiques). Le filtrat est ensuite évaporé. Du sel zwitterionique d'imidazolium dissymétrique est alors formé. En considérant la formule générale 1A : A⁻ est une charge négative sur le groupement R2.

Par ce protocole opératoire, les Demanderesses ont pu synthétiser des sels zwitterioniques d'imidazolium dissymétrique énantiomèriquement purs de formule 19 et 20 :

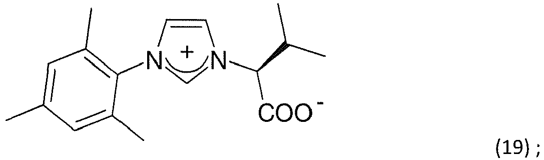

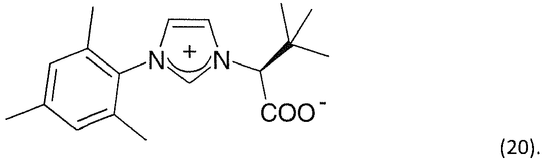

### EXEMPLES DE RÉALISATION

### 1. SYNTHÈSE D'UN SEL 1-ARYL-3-CYCLOALKYL-IMIDAZOLIUM - BF₄⁻:

Dans un ballon on place de l'aniline (Mesitylamine, 40 mmoles, 1 eq) et la cycloalkylamine (40mmoles, 1eq). Puis de l'acide acétique (10 mL, 18 mmoles, 4.5 eq) est additionné lentement. Le mélange est ensuite laissé sous agitation pendant 5 minutes.

Dans un ballon sont placés du glyoxal (4.6 mL, 40 mmoles, 1 eq), du formol (3.0 mL, 40 mmoles, 1 eq) et de l'acide acétique (10 mL, 18 mmoles, 4.5 eq), puis le mélange est chauffé à 80°C. À cette solution est ensuite ajouté, goutte à goutte, le mélange d'amines préalablement préparé, puis le milieu est laissé à 80°C pendant le temps impartis (de 2h à 14h).

Une fois que la réaction est terminée, le milieu réactionnel est refroidi à température ambiante puis de l'eau (20mL) est additionnée, suivis de 40 mL de l'acétate d'éthyle (AcOEt). La phase aqueuse est extraite trois fois avec 20mL de l'acétate d'éthyle (AcOEt) puis les phases organiques sont combinées et séchées sur du sulfate de magnésium (MgSO₄), et concentrée sous vide. L'analyse par résonnance magnétique nucléaire (RMN) du brut réactionnel permet de déterminer la sélectivité de la réaction.

Le brut réactionnel est ensuite dissous dans 70 mL de dichlorométhane (CH₂Cl₂) puis 5.15 g de tetrafluoroborate de potassium (KBF₄) (40 mmoles, 1 eq) sont ensuite ajouté. Le mélange est ensuite agité à température ambiante pendant 3h. Ensuite, on réalise une extraction liquide/liquide, à savoir une extraction eau/solvant organique (ici H₂O/CH₂Cl₂). Pour cela, 20mL d'eau sont ajouté, les phases sont séparées, la phase aqueuse est lavée trois fois avec 20mL de dichlorométhane (CH₂Cl₂). Les phases organiques sont ensuite combinées, séchées sur sulfate de magnésium (MgSO₄), et concentrées sous vide. À l'huile marron résultante est ensuite ajouté 40mL d'acétate d'éthyle (AcOEt) puis le mélange est placé aux ultrasons pendant 5 minutes. Un solide se forme lequel est filtré sur fritté, puis lavé avec de l'acétate d'éthyle (AcOEt) pour donner l'imidazolium 1-aryl-3-cycloalkyl souhaité.

Chaque sel d'imidazolium 1-aryl-3-cycloalkyl souhaité a été soumis à une analyse RMN et a été confirmé par analyse cristallographique. Des données d'analyse RMN sont fournis ci-dessous pour différents sels d'imidazolium 1-aryl-3-cycloalkyl.

Les spectres de RMN ¹H (400MHz), ¹³C (125MHz), ³¹P (162MHz), ¹¹B (128MHz) et ¹⁹F (376 MHz) ont été réalisés sur un spectromètre à transformée de Fourier Brucker ARX 400 avec découplage du proton pour tous les noyaux excepté ¹H. Les déplacements chimiques (δ) sont exprimés en partie par million (ppm), dans le solvant deutérié indiqué. Les abréviations suivantes ont été utilisées pour désigner la multiplicité des signaux s (singulet), d (doublet), t (triplet), q (quadruplet), quin. (quintuplet), sept. (septuplet), m (multiplet), bs (singulet large).

### la. 5-CYCLOPENTYL -2-MESITYL-IMIDAZOLIUM TETRAFLUOROBORATE

| | |
|---|---|
| 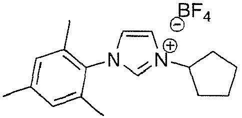 | Formule brute : C₁₇H₂₃BF₄N₂ |
| | M = 342.18 g/mol |
| | Rendement : 35% |

L'utilisation de la procédure générale pour la préparation d'imidazolium dissymétrique avec 3.41g (40 mmoles) de cyclopentylamine et 5.6mL de mésitylamine (40 mmoles) conduit à 4.80 g (14 mmoles, 35%) d'imidazolium dissymétrique sous la forme d'un solide blanc.

### Sélectivité de la réaction :

| | | |
|---|---|---|
| 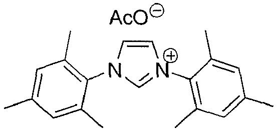 | 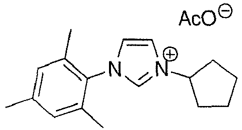 | 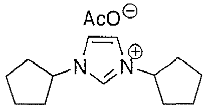 |
| 1 partie | 28.2 parties | 1.1 partie |

¹H (400MHz, CDCl₃) : 8,82 (t, *J* = 1,73Hz, 1Hᵢₘ) ; 7,70 (t, *J* = 1,7Hz, 1Hᵢₘ) ; 7,24 (t, *J* = 1,7 Hz, 1Hᵢₘ) ; 6,97 (s, 2Hₐᵣ) ; 5,03 (q, *J* = 7,5Hz, 1H) ; 2,42 (m, 2H) ; 2,32 (s, 3Hₘₑₛ); 2,00 (s, 6Hₘₑₛ); 1,92 (m, 4H); 1,77 (m, 2H)
¹³C (125 MHz, CDCl₃) : 141.31 ; 136.6; 134.4 (2Cₘₑₛ); 130.8; 129.9 (2Cₘₑₛ) ; 124.3 (Cᵢₘ); 121.4(Cᵢₘ); 62.0; 35.6 (2C); 24.0 (2C); 21.2; 17.3 (2Cₘₑₛ)
¹⁹F (376 MHz, CDCl₃) : -151.98 / -152,0 (s)
¹¹B (138 MHz, CDCl₃) : -1.033 (s, BF₄)
Tf (température de fusion) : 96°C
HRMS (spectrospcopie de masse haute resolution) [M⁺] :
Calculée : 255.18612 Trouvée 255.1861.

### 1b. 5-CYCLOHEXYL -2-MESITYL-IMIDAZOLIUM TETRAFLUOROBORATE

| | |
|---|---|
| 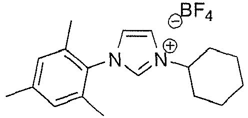 | Formule brute : C₁₈H₂₅BF₄N₂ |
| | M = 356.21 g/mol |
| | Rendement : 54% |

L'utilisation de la procédure générale pour la préparation d'imidazolium dissymétrique avec 4.6 mL (40 mmoles) de cyclohexylamine et 5.6 mL de mésitylamine (40 mmoles) conduit à 7.79 g (21.8 mmoles, 54%) d'imidazolium dissymétrique sous la forme d'un solide blanc.

### Sélectivité de la réaction :

| | | |
|---|---|---|
| 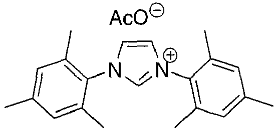 | 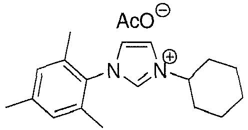 | 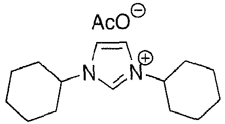 |
| 1 partie | 28.3 parties | 1 partie |

¹H (400MHZ, CDCl₃) : 8.85 (t, *J* = 1,8Hz, 1Hᵢₘ) ; 7.73 (t, *J* = 1,8Hz, 1Hᵢₘ) ; 7.22 (t, *J* = 1,8 Hz, 1Hᵢₘ) ; 6.97 (s, 2Hₐᵣ) ; 4.58 (m, 1H) ; 2.32 (s, 3Hₘₑₛ); 2.21 (m, 2H); 2.00 (s, 6Hₘₑₛ); 1.89 (m, 2H); 1.75 (m, 3H); 1.51 (m, 2H); 1.29 (m, 1H)
¹³C (125 MHz, CDCl₃) : 141.3 ; 135.7; 134.4 (2Cₘₑₛ) ; 130.9 ; 129.9 (2Cₘₑₛ) ; 123.9 (Cᵢₘ); 121.1(Ciₘ); 60.5; 33.6 (2C); 25.0 (2C); 24.7 ;21.2; 17.3 (2Cₘₑₛ)
¹⁹F (376 MHz, CDCl₃) : -151.74 (s)
¹¹B (138 MHz, CDCl₃) : -0.992 (s, BF₄)
Tf : 170 °C
HRMS [M⁺] : Calculée : 269.20177 Trouvée 269.2019.

### 1c. 5-CYCLOHEPTYL -2-MESITYL-IMIDAZOLIUM TETRAFLUOROBORATE

| | |
|---|---|
| 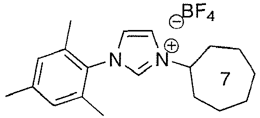 | Formule brute : C₁₉H₂₇BF₄N₂ |
| | M = 370.24 g/mol |
| | Rendement : 68% |

L'utilisation de la procédure générale pour la préparation d'imidazolium dissymétrique avec 2.55 mL (20 mmoles) de cycloheptylamine et 2.8 mL de mésitylamine (20 mmoles) conduit à 5.03 g (13.6 mmoles, 68%) d'imidazolium dissymétrique sous la forme d'un solide blanc.

### Sélectivité de la réaction :

| | | |
|---|---|---|
| 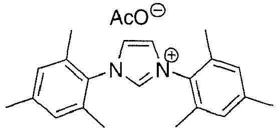 | 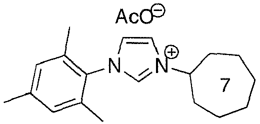 | 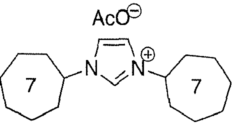 |
| 1 partie | 28.8 parties | 1.4 partie |

¹H (400MHz, CDCl₃) : 8.85 (t, *J* = 1,8Hz, 1Hᵢₘ) ; 7.72 (t, *J* = 1,8Hz, 1Hᵢₘ) ; 7.22 (t, *J* = 1,8 Hz, 1Hᵢₘ) ; 6.97 (s, 2Hₐᵣ) ; 4.76 (m, 1H) ; 2.31 (s, 3Hₘₑₛ); 2.21 (m, 2H); 1.99 (m, 2H + 6Hₘₑₛ); 1.80 (m, 2H); 1.62 (m, 6H)
¹³C (125 MHz, CDCl₃) : 141.2 ; 135.5; 134.4 (2Cₘₑₛ); 130.9; 129.8 (2Cₘₑₛ) ; 124.1 (Cᵢₘ); 121.1(Cᵢₘ); 62.8; 36.0 (2C); 27.0 (2C); 24.1 (2C) ; 21.2 ; 17.3 (2Cₘₑₛ)
¹⁹F (376 MHz, CDCl₃) : -151,6 (s)
¹¹B (138 MHz, CDCl₃) : -0.979 (s, BF₄)
Tf : 185°C
HRMS [M⁺]: Calculée : 283.21742 Trouvée : 283.2173.

### 1d. 5-CYCLOOCTYL -2-MESITYL-IMIDAZOLIUM TETRAFLUOROBORATE

| | |
|---|---|
| 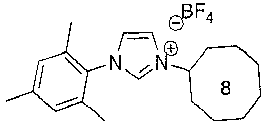 | Formule brute : C₂₀H₂₉BF₄N₂ |
| | M = 384.26 g/mol |
| | Rendement : 43% |

L'utilisation de la procédure générale pour la préparation d'imidazolium dissymétrique avec 5.6 mL (40 mmoles) de cyclooctylamine et 5.6 mL de mésitylamine (40 mmoles) conduit à 6.61 g (17.2 mmoles, 43%) d'imidazolium dissymétrique sous la forme d'un solide blanc.

### Sélectivité de la réaction :

| | | |
|---|---|---|
| 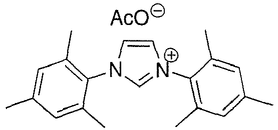 | 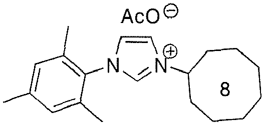 | 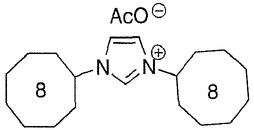 |
| 1 partie | 28.7 parties | 1.6 partie |

¹H (400MHz, CDCl₃) : 8.86 (t, *J* = 1,7Hz, 1Hᵢₘ) ; 7.70 (t, *J* = 1,7Hz, 1Hᵢₘ) ; 7.23 (t, *J* = 1,7 Hz, 1Hᵢₘ) ; 6.97 (s, 2Hₐᵣ) ; 4.58 (q, *J* = 6.8Hz, 1H) ; 2.31 (s, 3Hₘₑₛ); 2.11 (m, 4H); 2.00 (s, 6Hₘₑₛ); 1.63 (m, 10H)
¹³C (125 MHz, CDCl₃) : 141.2 ; 135.5; 134.4 (2Cₘₑₛ); 130.9 ; 129.9 (2Cₘₑₛ) ; 124.1 (Cᵢₘ); 121.3(Cᵢₘ); 62.1; 33.9 (2C); 26.3 (2C); 25.5 ; 23.9 ; 21.2; 17.3 (2Cₘₑₛ)
¹⁹F (376 MHz, CDCl₃) : -151,6 (s)
¹¹B (138 MHz, CDCl₃) : -0,990 (s, BF₄)
Tf : 175°C
HRMS [M⁺]: Calculée : 297.23307 Trouvée : 297.2332.

### 1e. 5-CYCLODODECYL-2-MESITYL-IMIDAZOLIUM TETRAFLUOROBORATE

| | |
|---|---|
| 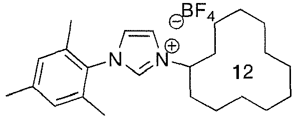 | Formule brute : C₂₄H₃₇BF₄N₂ |
| | M = 440.37 g/mol |
| | Rendement : 62% |

L'utilisation de la procédure générale pour la préparation d'imidazolium dissymétrique avec 7.6 mL (40 mmoles) de cyclododecylamine et 5.6 mL de mésitylamine (40 mmoles) conduit à 10.90 g (24.8 mmoles, 62%) d'imidazolium dissymétrique sous la forme d'un solide blanc.

### Sélectivité de la réaction :

| | | |
|---|---|---|
| 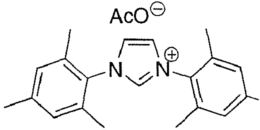 | 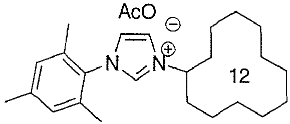 | 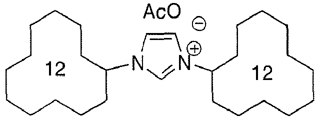 |
| 1 partie | 30 parties | 1 partie |

¹H (400MHz, CDCl₃) : 8.86 (t, J = 1,8Hz, 1Hᵢₘ) ; 7.73 (t, J = 1,8Hz, 1Hᵢₘ) ; 7.27 (t, J = 1,8 Hz, 1Hᵢₘ) ; 6.96 (s, 2Hₐᵣ) ; 4.74 (m, 1H) ; 2.31 (s, 3Hₘₑₛ); 2.11 (m, 2H); 2.00 (s, 6Hₘₑₛ); 1.83 (m, 2H); 1.40 (m, 18H)
¹³C (125 MHz, CDCl₃) : 141.1 ; 135.9; 134.4 (2Cₘₑₛ) ; 130.9; 129.8 (2Cₘₑₛ) ; 124.2 (Cᵢₘ); 121.9(Cᵢₘ); 59.4; 30.2 (2C); 23.7 ;23.4 (2C) ;23.35(2C) ;23.3(2C) ;21.4(2C) ; 21.2; 17.2 (2Cₘₑₛ)
¹⁹F (376 MHz, CDCl₃) : -151.4 / -151,6 (s)
¹¹B (138 MHz, CDCl₃) : -1.033 (s, BF₄)
Tf : 177°C
HRMS [M⁺]: Calculée : 353.29567 Trouvée 353.2956.

### 1f. 5-CYCLODODECYL-2-(3-5-DIMETHYLPHENOL)-IMIDAZOLIUM TETRAFLUOROBORATE

| | |
|---|---|
| 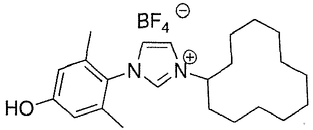 | Formule brute : C₂₃H₃₅BF₄N₂O |
| | M = 442.34 g/mol |
| | Rendement : 70% |

L'utilisation de la procédure générale pour la préparation d'imidazolium dissymétrique avec 1.37 g (10 mmol ; 1eq.) de 4-amino-3,5-dimethylphenol et 1.83g (10 mmol, 1eq.) de cyclododecylamine conduit à 3.10 g (7 mmol, 70%) de produit escompté sous la forme d'un solide marron.

### Sélectivité de la réaction :

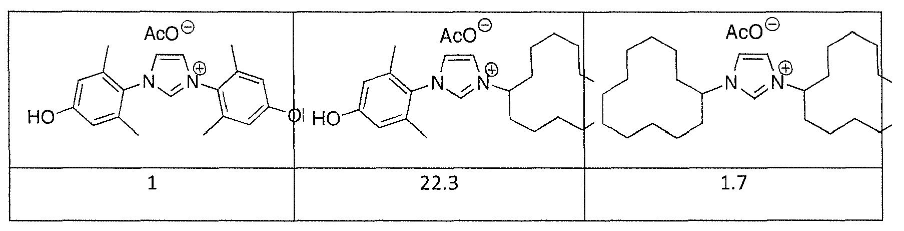

¹H (400 MHz, CD₃OD) : 9.39 (s, 1H), 8.06 (d, ³*J*_{H-H} = 1.98 Hz,1H), 7.72 (d, ³*J*_{H-H} = 1.98 Hz,1H), 6.70 (s, 2H), 4.72 (m,1H), 2.27-2.16 (m, 2H), 2.03 (s, 6H), 1.94-1.84 (m, 2H), 1.63-1.32 (m, 18 H)
¹⁹F (128 MHz, CDCl₃) : -154.4 (s)
¹¹B (400 MHz, CD₃OD) : -1.2 (s)

### 2. SYNTHÈSE DU SEL ZWITTERIONIQUE (S)-2-(MESITYLIMIDAZOLIUM)-3-METHYLBUTANOATE :

L'utilisation de la procédure générale pour l'obtention de sels d'imidazolium dissymétriques avec 1.17 g de (L)-valine (10 mmoles, 1equiv.), 1.4 mL (10 mmoles, 1 equiv.) de mesitylamine permet l'obtention du sel d'acétate.

Dans un ballon on place 1.17 g de (L)-valine (10 mmoles, 1 eq), et 1.4 mL (10 mmoles, 1 eq) de mesitylamine. Puis de l'acide acétique (10 mL, 18 mmoles, 4.5 eq) est additionné lentement. Le mélange est ensuite laissé sous agitation pendant 5 minutes.

Dans un ballon sont placé du glyoxal (4.6 mL, 40 mmoles, 1 eq), du formol (3.0 mL, 40 mmoles, 1 eq) et de l'acide acétique (10 mL, 18 mmoles, 4.5 eq), puis le mélange est chauffé à 80°C. À cette solution est ensuite ajouté, goutte à goutte, le mélange valine/mesitylamine préalablement préparé, puis le milieu est laissé à 80°C pendant le temps impartis (de 2h à 14h).

Une fois que la réaction est terminée, le milieu réactionnel est refroidi à température ambiante puis de l'eau (20mL) est additionnée, suivis de 40mL de l'acétate d'éthyle (AcOEt).

Après la dissolution du brut réactionnel dans de l'acétate d'éthyle (AcOEt) (10mL) la phase organique est lavée deux fois avec 5 mL d'une solution saturée de bicarbonate de sodium (NaHCO₃) puis les phases aqueuses sont combinées puis évaporées conduisant à un solide jaune. Le solide est ensuite repris dans de l'acétone, puis filtré sur fritté (pour enlever les sels inorganiques). Le filtrat est ensuite évaporé pour donner 1.00 g (3.5 mmoles, 35%) de (S)-2-(mesitylimidazolium)-3-methylbutanoate sous la forme d'un solide jaunâtre.

Le sel zwitterionique (S)-2-(mesitylimidazolium)-3-methylbutanoate a été soumis à une analyse RMN et a été confirmé par analyse cristallographique. Des données d'analyse RMN sont fournis ci-dessous (les abréviations et conditions étant similaires à ce qui est décrit plus haut) :
¹H (400MHz, CDCl₃) : 9.28 (s, 1H); 7.84 (bs, 1H); 7.08 (bs, 1H); 6.83 (bs, 2H); 4.66 (d, J = 7.5 Hz, 1H); 2.34 (hex., J = 6.7Hz, 1H); 2.17 (bs, 3H); 1.87 (bs, 6H); 0.85 (d, J = 6.7Hz, 3H); 0.69 (d, J = 6.7 Hz, 3H).
¹³C (125 MHz, CDCl₃) : 174.6 ; 170.4 ; 141.5 ; 137.1 ; 131.0 ; 130.0 ; 124.3 ; 121.5 ; 73.0 ; 32.6 ; 21.7; 21,2 ; 19.9 ; 18.6.
HRMS [M+]: Calculée : 287.17595 Trouvée 287.1757.

### 3. SYNTHÈSE DU SEL ZWITTERIONIQUE (S)-2-(MESITYLIMIDAZOLIUM)-3,3-DIMETHYLBUTANOATE

L'utilisation de la procédure générale pour l'obtention de sels d'imidazolium dissymétrique avec 0.262g (0.2 mmoles, 1 eq) de *ter*-Leucine, 0.28 mL (0.2 mmoles, 1 eq) de mésitylamine permet l'obtention du sel d'acétate.

Dans un ballon on place 0.262g (0.2 mmoles, 1 eq) de *ter*-Leucine, et 0.28 mL (0.2 mmoles, 1 eq) de mesitylamine. Puis de l'acide acétique (10 mL, 18 mmoles, 4.5 eq) est additionné lentement. Le mélange est ensuite laissé sous agitation pendant 5 minutes.

Dans un ballon sont placé du glyoxal (4.6 mL, 40 mmoles, 1 eq), du formol (3.0 mL, 40 mmoles, 1 eq) et de l'acide acétique (10 mL, 18 mmoles, 4.5 eq), puis le mélange est chauffé à 80°C. À cette solution est ensuite ajouté, goutte à goutte, le mélange valine/mesitylamine préalablement préparé, puis le milieu est laissé à 80°C pendant le temps impartis (de 2h à 14h).

Une fois que la réaction est terminée, le milieu réactionnel est refroidi à température ambiante puis de l'eau (20mL) est additionnée, suivis de 40mL de l'acétate d'éthyle (AcOEt).

Après la dissolution du brut réactionnel dans de l'AcOEt (3mL) la phase organique est lavée deux fois avec une solution saturée de NaHCO₃ puis les phases aqueuses sont combinée puis évaporée conduisant à un solide jaune. Le solide est ensuite repris dans de l'acétone, puis filtré sur fritté. Le filtrat est ensuite évaporé pour donner 0.106 g (0.035 mmoles, 17%) de (S)-2-(mésitylimidazolium)-3,3-dimethylbutanoate sous la forme d'un solide blanc.

Le sel zwitterionique ((S)-2-(mésitylimidazolium)-3,3-dimethylbutanoate a été soumis à une analyse RMN et a été confirmé par analyse cristallographique. Des données d'analyse RMN sont fournis ci-dessous (les abréviations et conditions étant similaires à ce qui est décrit plus haut) :
¹H (400MHz, CDCl₃) : 9.17 (s, 1H); 7.87 (s, 1H); 7.11 (s, 1H); 7.00 (s, 2H); 4.80 (s, 1H); 2.34 (s, 3H); 2.24 (bs; 2H); 2.00 (s, 6H); 1.00 (s, 9H).
¹⁹F (376 MHz, CDCl₃) : -71.43; -73.32
³¹P (162 MHz, CDCl₃) : -135.6 ; -140.0 ; -144.4 ; -148.8 ; -153.2
HRMS [M⁺]: Calculée : 301.19160 Trouvée : 301.1913.

Un avantage de la présente invention, à savoir le fait de synthétiser des sels d'imidazolium dissymétriques comportant des groupements R1 et R2 tels que décrits ci-dessus, est qu'ils confèrent une bonne stabilité (vraisemblablement en raison des interactions stériques importantes). Le carbène imidazoline-2-ylidene étant stable, ceci évite la réaction parasite de dimérisation de l'espèce carbénique sur elle-même comme décrit dans NHCs in Synthesis, S. P. Nolan, Ed., 2006, Wiley-VCH. De ce fait, de bons rendements pour la synthèse de complexes organométalliques cibles sont observés. La présente invention offre une voie inédite pour synthétiser des sels précurseurs de diaminocarbènes de type 1-aryl-3-cycloalkyl-imidazoline-2-ylidene ou 1-aryl-3-hétéroalkyl-imidazoline-2-ylidene.

Cette voie d'obtention inédite est rapide, efficace (1 seule opération chimique) permettant de générer des sels d'imidazolium 1,3-disubstitués dissymétriques portant un groupement aromatique d'une part, et un groupement cycloalkyle (ou hétéroalkyl) d'autre part.

## Revendications

1. Procédé de préparation d'un sel d'imidazolium dissymétrique intermédiaire de formule 1A : 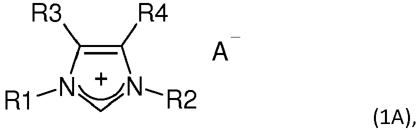 dans laquelle
R1 est un groupement aromatique,
R2 est choisi parmi un groupement alkyle aliphatique secondaire cyclique et un groupement hétéroalkyle,
R3 et R4 sont choisis indépendamment l'un de l'autre dans le groupe constitué d'hydrogène, d'halogénure et d'un groupement alkyle, et
A⁻ est un anion,
comprenant les étapes suivantes :
a. former un mélange réactionnel par la mise en contact d'un équivalent (1 eq) d'une aniline de formule 2 : 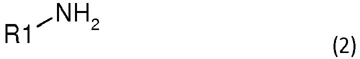 avec un équivalent (1 eq) d'un composé de formule 3 : 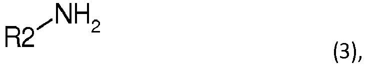 en présence d'au moins quatre virgule cinq équivalents (4,5 eq) d'un acide de Brønsted de formule 4 :
AH (4);
b. former une solution comprenant un équivalent (1 eq) d'un dicarbonyle de formule 5 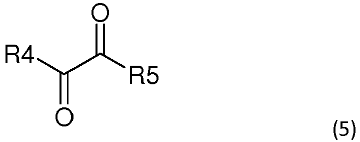 un équivalent (1 eq) de formaldéhyde, et au moins quatre virgule cinq équivalents (4,5 eq) de l'acide de Brønsted de formule 4, porter ladite solution à environ 80°C et y ajouter le mélange réactionnel formé à l'étape a. ;
c. laisser sous agitation pendant au moins 2 heures à environ 80°C ; et
d. isoler le sel d'imidazolium dissymétrique intermédiaire de formule 1A.

2. Procédé de préparation d'un sel d'imidazolium dissymétrique de formule 1B : 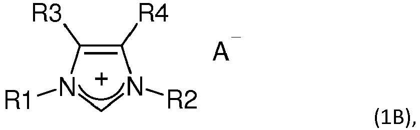 dans laquelle
R1 est un groupement aromatique,
R2 est choisi parmi un groupement alkyle aliphatique secondaire cyclique et un groupement hétéroalkyle,
R3 et R4 sont choisis indépendamment l'un de l'autre dans le groupe constitué d'hydrogène, d'halogénure et d'un groupement alkyle, et
A⁻ est choisi parmi le groupe constitué d'un anion tetrafluoroborate, un anion hexafluorophosphate un anion hexafluoroantimoine, un anion tetrakis[(3,5-trifluorométhyl)phényl]borate et un anion halogénure,
ledit procédé comprenant les étapes a. à d. selon la revendication 1, et comprenant en outre les étapes de :
e. ajouter un équivalent (1eq) d'un sel inorganique et du solvant, préférentiellement du dichlorométhane, au sel d'imidazolium dissymétrique intermédiaire isolé à l'étape d. ;
f. laisser sous agitation à température ambiante pendant au moins une heure et réaliser une extraction eau/solvant organique suivi d'une évaporation dudit solvant organique ;
g. précipiter par un solvant organique polaire, puis isoler le sel d'imidazolium dissymétrique de formule 1B.

3. Procédé de préparation d'un sel d'imidazolium dissymétrique selon la revendication 2, dans lequel le sel inorganique de l'étape e. est choisi parmi le groupe constitué de tetrafluoroborate de potassium, de tetrafluoroborate de sodium, de tetrafluoroborate de lithium, de tetrafluoroborate d'hydrogène, de tetrafluoroborate d'ammonium, de hexafluorophosphate de potassium, de hexafluorophosphate de sodium, de hexafluorophosphate de lithium, de hexafluorophosphate d'hydrogène, de hexafluorophosphate d'ammonium, d'hexafluoroantimoine d'argent, d'hexafluoroantimoine de potassium, d'hexafluoroantimoine de sodium, d'hexafluoroantimoine de lithium, de tetrakis[(3,5-trifluorométhyl)phényl]borate de potassium, de tetrakis[(3,5-trifluorométhyl)phényl]borate de sodium et de tetrakis[(3,5-trifluorométhyl)phényl]borate de lithium et d'acide halogénique.

4. Procédé de préparation d'un sel d'imidazolium dissymétrique de formule 1C : 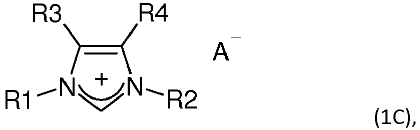 dans laquelle
R1 est un groupement aromatique,
R2 est un groupement hétéroalkyle,
R3 et R4 sont choisis indépendamment l'un de l'autre dans le groupe constitué d'hydrogène, d'halogénure et d'un groupement alkyle, et
A⁻ est une charge négative sur le groupement R2,
ledit procédé comprenant les étapes a. à d. selon la revendication 1, et comprenant en outre les étapes de :
h. ajouter au moins dix équivalents (10 eq) d'une base carbonatée au sel d'imidazolium dissymétrique intermédiaire isolé à l'étape d. ;
i. isoler le sel d'imidazolium dissymétrique de formule 1C.

5. Procédé de préparation d'un sel d'imidazolium dissymétrique selon la revendication 4, dans lequel ladite base carbonatée de l'étape h. est choisie dans le groupe constitué de bicarbonate de sodium, de bicarbonate de potassium, d'hydrogénocarbonate de sodium et d'hydrogénocarbonate de potassium.

6. Procédé selon l'une des revendications précédentes, dans lequel l'acide de Brønsted de formule 4 desdites étapes a. et b. est l'acide acétique.

7. Procédé selon l'une des revendications précédentes, dans lequel R1 est choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle.

8. Procédé selon l'une des revendications 1 à 3, 6 et 7, dans lequel R2 est choisi dans le groupe constitué de cyclopentyle, de cyclohexyle, de cyclooctyle, de cyclodecyle, de cyclododecyle, et de cyclopentadecyle.

9. Procédé selon l'une des revendications 4 et 5, dans lequel R2 est choisi dans le groupe constitué de 3-methylbutanoate et 3,3-dimethylbutanoate.

10. Procédé selon l'une des revendications précédentes, dans lequel R3 et R4 sont chacun l'hydrogène.

11. Sel d'imidazolium dissymétrique de formule 1D obtenu par le procédé selon l'une des revendications précédentes, 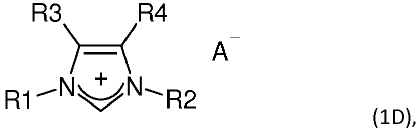 dans lequel
R1 est un groupement aromatique choisi dans le groupe constitué de 2,4,6-triméthylphényle, de 3,5-dinitrophényle, de 2,4,6-tris(trifluorométhyle)phényle, de 2,4,6-trichlorophényle, et de hexafluorophényle,
R2 est un groupement alkyle aliphatique secondaire cyclique,
R3 et R4 sont choisis indépendamment l'un de l'autre dans le groupe constitué d'hydrogène, d'halogénure et d'un groupement alkyle, et
A⁻ est un anion choisi parmi le groupe constitué d'un anion tetrafluoroborate, d'un anion hexafluorophosphate, d'un anion acétate, d'un anion hexafluoroantimoine, d'un anion tetrakis[(3,5-trifluorométhyl)phényl]borate et d'un anion halogénure.

12. Sel d'imidazolium dissymétrique selon la revendication 11, dans lequel R2 est choisi dans le groupe constitué de cyclopentyle, de cyclohexyle, de cycloheptyle, de cyclooctyle, de cyclodecyle, de cyclododecyle, et de cyclopentadecyle.

13. Sel d'imidazolium dissymétrique selon l'une des revendications 11 et 12, dans lequel R3 et R4 sont chacun l'hydrogène.

## Patentansprüche

1. Verfahren zur Herstellung eines intermediären unsymmetrischen Imidazoliumsalzes der Formel 1A: 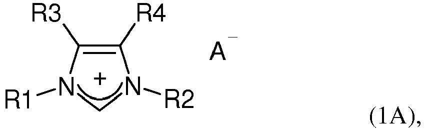 in der
R1 eine aromatische Gruppe ist,
R2 aus einer cyclischen sekundären aliphatischen Alkylgruppe und einer Heteroalkylgruppe ausgewählt ist,
R3 und R4 unabhängig voneinander aus der Gruppe ausgewählt sind, bestehend aus Wasserstoff, Halogen und einer Alkylgruppe, und
A⁻ ein Anion ist,
umfassend die folgenden Schritte:
a) Bilden einer Reaktionsmischung durch Inkontaktbringen eines Äquivalents (1 eq) eines Anilins der Formel 2: 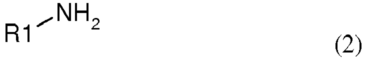 mit einem Äquivalent (1 eq) einer Verbindung der Formel 3: 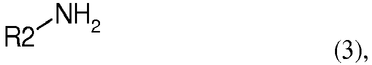 in Gegenwart von wenigstens 4,5 Äquivalenten (4,5 eq) einer Brönsted-Säure der Formel 4:
AH (4) ;
b) Bilden einer Lösung umfassend ein Äquivalent (1 eq) eines Dicarbonyls der Formel 5 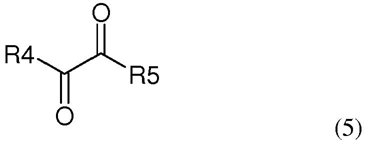 ein Äquivalent (1 eq) Formaldehyd und wenigstens 4,5 Äquivalente (4,5 eq) Brönsted-Säure der Formel 4, Halten besagter Lösung bei ungefähr 80°C und Zugabe der in Schritt a) gebildeten Reaktionsmischung;
c) wenigstens 2 Stunden Rühren bei ungefähr 80°C; und
d) Isolieren des intermediären unsymmetrischen Imidazoliumsalzes der Formel 1A.

2. Verfahren zur Herstellung eines unsymmetrischen Imidazoliumsalzes der Formel 1B: 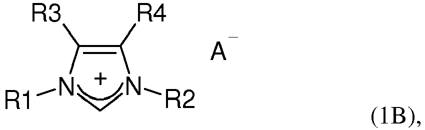 in der
R1 eine aromatische Gruppe ist,
R2 aus einer cyclischen sekundären aliphatischen Alkylgruppe und einer Heteroalkylgruppe ausgewählt ist,
R3 und R4 unabhängig voneinander aus der Gruppe ausgewählt sind, bestehend aus Wasserstoff, Halogen und einer Alkylgruppe, und
A⁻ aus der Gruppe ausgewählt ist, bestehend aus einem Tetrafluorborat-Anion, einem Hexafluorphosphat-Anion, einem Hexafluorantimonat-Anion, einem Tetrakis[(3,5-trifluormethyl)phenyl]borat-Anion und einem Halogenid-Anion,
wobei besagtes Verfahren die Schritte a) bis d) gemäß Anspruch 1 und außerdem die Schritte umfasst:
e) Zugabe eines Äquivalents (1 eq) eines anorganischen Salzes und des Lösemittels, vorzugsweise Dichlormethan, zu dem in Schritt d) isolierten intermediären unsymmetrischen Imidazoliumsalz;
f) Rühren bei Raumtemperatur über wenigstens eine Stunde und Durchführen einer Extraktion mit Wasser/organischem Lösemittel danach Verdampfen besagten organischen Lösemittels;
g) Fällen mit einem polaren organischen Lösemittel, dann Isolieren des unsymmetrischen Imidazoliumsalzes der Formel 1B.

3. Verfahren zur Herstellung eines unsymmetrischen Imidazoliumsalzes gemäß Anspruch 2, wobei das anorganische Salz von Schritt e) aus der Gruppe ausgewählt ist, bestehend aus Kaliumtetrafluorborat, Natriumtetrafluorborat, Lithiumtetrafluorborat, Hydrogentetrafluorborat, Ammoniumtetrafluorborat, Kaliumhexafluorphosphat, Natriumhexafluorphosphat, Lithiumhexafluorphosphat, Hydrogenhexafluorphosphat, Ammoniumhexafluorphosphat, Silberhexafluorantimonat, Kaliumhexafluorantimonat, Natriumhexafluorantimonat, Lithiumhexafluorantimonat, Kaliumtetrakis[(3,5-trifluormethyl)phenyl]borat, Natriumtetrakis[(3,5-trifluormethyl)phenyl]borat und Lithiumtetrakis[(3,5-trifluormethyl)phenyl]borat und Halogensäure.

4. Verfahren zur Herstellung eines unsymmetrischen Imidazoliumsalzes der Formel 1C: 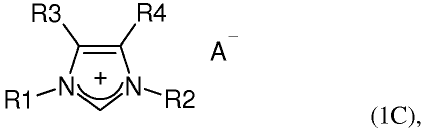 in der
R1 eine aromatische Gruppe ist,
R2 eine Heteroalkylgruppe ist,
R3 und R4 unabhängig voneinander aus der Gruppe ausgewählt sind, bestehend aus Wasserstoff, Halogen und einer Alkylgruppe, und
A⁻ eine negative Ladung auf der Gruppe R2 ist,
wobei besagtes Verfahren die Schritte a) bis d) gemäß Anspruch 1 umfasst und außerdem die Schritte umfasst:
h) Zugabe von wenigstens 10 Äquivalenten (10 eq) einer carbonathaltigen Base zu dem in Schritt d) isolierten intermediären unsymmetrischen Imidazoliumsalz;
i) Isolieren des unsymmetrischen Imidazoliumsalzes der Formel 1C.

5. Verfahren zur Herstellung eines unsymmetrischen Imidazoliumsalzes gemäß Anspruch 4, wobei besagte carbonathaltige Base von Schritt h) aus der Gruppe ausgewählt ist, bestehend aus Natriumbicarbonat, Kaliumbicarbonat, Natriumhydrogencarbonat und Kaliumhydrogencarbonat.

6. Verfahren gemäß einem der vorhergehenden Ansprüche, wobei die Brönsted-Säure der Formel 4 besagter Schritte a) und b) Essigsäure ist.

7. Verfahren gemäß einem der vorhergehenden Ansprüche, wobei R1 aus der Gruppe ausgewählt ist, bestehend aus 2,4,6-Trimethylphenyl, 3,5-Dinitrophenyl, 2,4,6-Tris(trifluormethyl)phenyl, 2,4,6-Trichlorphenyl und Hexafluorphenyl.

8. Verfahren gemäß einem der Ansprüche 1 bis 3, 6 und 7, wobei R2 aus der Gruppe ausgewählt ist, bestehend aus Cyclopentyl, Cyclohexyl, Cyclooctyl, Cyclodecyl, Cyclododecyl und Cyclopentadecyl.

9. Verfahren gemäß einem der Ansprüche 4 und 5, wobei R2 aus der Gruppe ausgewählt ist, bestehend aus 3-Methylbutanoat und 3,5-Dimethylbutanoat.

10. Verfahren gemäß einem der vorhergehenden Ansprüche, wobei R3 und R4 jeweils Wasserstoff sind.

11. Unsymmetrisches Imidazoliumsalz der Formel 1D, erhalten mit dem Verfahren gemäß einem der vorhergehenden Ansprüche, 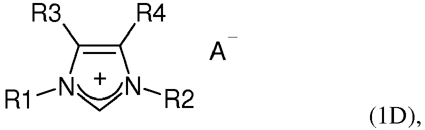 in der
R1 eine aromatische Gruppe ist, ausgewählt aus der Gruppe, bestehend aus 2,4,6-Trimethylphenyl, 3,5-Dinitrophenyl, 2,4,6-Tris(trifluormethyl)phenyl, 2,4,6-Trichlorphenyl und Hexafluorphenyl,
R2 eine cyclische sekundäre aliphatische Alkylgruppe ist,
R3 und R4 unabhängig voneinander aus der Gruppe ausgewählt sind, bestehend aus Wasserstoff, Halogen und einer Alkylgruppe, und
A⁻ ein Anion ist, ausgewählt aus der Gruppe, bestehend aus einem Tetrafluorborat-Anion, einem Hexafluorphosphat-Anion, einem Acetat-Anion, einem Hexafluorantimonat-Anion, einem Tetrakis[(3,5-trifluormethyl)phenyl]borat-Anion und einem Halogenid-Anion.

12. Unsymmetrisches Imidazoliumsalz gemäß Anspruch 11, wobei R2 aus der Gruppe ausgewählt ist, bestehend aus Cyclopentyl, Cyclohexyl, Cycloheptyl, Cyclooctyl, Cyclodecyl, Cyclododecyl und Cyclopentadecyl.

13. Unsymmetrisches Imidazoliumsalz gemäß einem der Ansprüche 11 und 12, wobei R3 und R4 jeweils Wasserstoff sind.

## Claims

1. Method of preparing an intermediate asymmetric imidazolium salt of formula 1A: 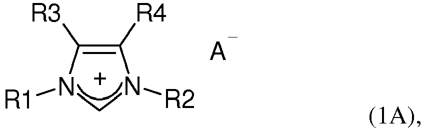 wherein
R1 is an aromatic group,
R2 is selected from a secondary cyclic aliphatic alkyl group and a heteroalkyl group,
R3 and R4 are selected independently of one another from the group consisting of
hydrogen, a halide and an alkyl group, and
A⁻ is an anion,
comprising the following steps:
a. forming a reaction mixture by contacting one equivalent (1 eq) of an aniline of formula 2 : 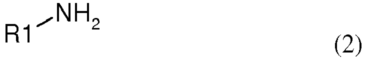 with one equivalent (1 eq) of a compound of formula 3 : 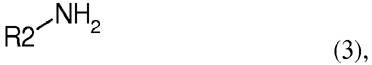 in the presence of at least four point five equivalents (4.5 eq) of a Brønsted acid of formula 4 :
AH (4);
b. forming a solution comprising one equivalent (1 eq) of a dicarbonyl of formula 5 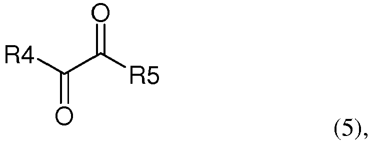 one equivalent (1 eq) of formaldehyde, and at least four point five equivalents (4.5 eq) of the Brønsted acid of formula 4, heating said solution to about 80°C and then adding the reaction mixture formed in step a.;
c. stirring for at least 2 hours at about 80°C; and
d. isolating the intermediate asymmetric imidazolium salt of formula 1A.

2. Method of preparing an asymmetric imidazolium salt of formula 1B: 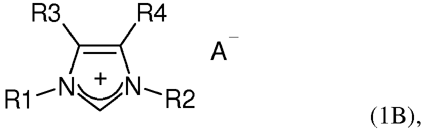 wherein
R1 is an aromatic group,
R2 is selected from a secondary cyclic aliphatic alkyl group and a heteroalkyl group, R3 and R4 are selected independently of one another from the group consisting of hydrogen, a halide and an alkyl group, and
A⁻ is selected from the group consisting of a tetrafluoroborate anion, a hexafluorophosphate anion, a hexafluoroantimony anion, a tetrakis[(3,5-trifluoromethyl)phenyl]borate anion and a halide anion,
said method comprising steps a. to d. according to Claim 1, and further comprising the steps of:
e. adding one equivalent (1eq) of an inorganic salt and solvent, preferably dichloromethane, to the intermediate asymmetric imidazolium salt isolated in step d.;
f. stirring at room temperature for at least one hour and carrying out water/organic solvent extraction followed by evaporation of said organic solvent;
g. precipitating by a polar organic solvent, then isolating the asymmetric imidazolium salt of formula 1B.

3. Method of preparing an asymmetric imidazolium salt according to Claim 2, wherein the inorganic salt in step e. is selected from the group consisting of potassium tetrafluoroborate, sodium tetrafluoroborate, lithium tetrafluoroborate, hydrogen tetrafluoroborate, ammonium tetrafluoroborate, potassium hexafluorophosphate, sodium hexafluorophosphate, lithium hexafluorophosphate, hydrogen hexafluorophosphate, ammonium hexafluorophosphate, silver hexafluoroantimony, potassium hexafluoroantimony, sodium hexafluoroantimony, lithium hexafluoroantimony, potassium tetrakis[(3,5-trifluoromethyl)phenyl]borate, sodium tetrakis[(3,5-trifluoromethyl)phenyl]borate and lithium tetrakis[(3,5-trifluoromethyl)phenyl]borate and halogen acid.

4. Method of preparing an asymmetric imidazolium salt of formula 1C: 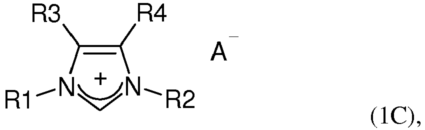 wherein
R1 is an aromatic group,
R2 is a heteroalkyl group,
R3 and R4 are selected independently of one another from the group consisting of hydrogen, a halide and an alkyl group, and
A⁻ is a negative charge on group R2,
said method comprising steps a. to d. according to Claim 1, and further comprising the steps of:
h. adding at least ten equivalents (10 eq) of a carbonate base to the intermediate asymmetric imidazolium salt isolated in step d.;
i. isolating the asymmetric imidazolium salt of formula 1C.

5. Method of preparing an asymmetric imidazolium salt according to Claim 4, wherein said carbonate base in step h. is selected from the group consisting of sodium bicarbonate, potassium bicarbonate, sodium hydrogen carbonate and potassium hydrogen carbonate.

6. Method according to one of the preceding claims, wherein the Brønsted acid of formula 4 in said steps a. and b. is acetic acid.

7. Method according to one of the preceding claims, wherein R1 is selected from the group consisting of 2,4,6-trimethylphenyl, 3,5-dinitrophenyl, 2,4,6-tris(trifluoromethyl)phenyl, 2,4,6-trichlorophenyl, and hexafluorophenyl.

8. Method according to one of Claims 1 to 3, 6 and 7, wherein R2 is selected from the group consisting of cyclopentyl, cyclohexyl, cyclooctyl, cyclodecyl, cyclododecyl, and cyclopentadecyl.

9. Method according to one of Claims 4 and 5, wherein R2 is selected from the group consisting of 3-methyl butanoate and 3,3-dimethyl butanoate.

10. Method according to one of the preceding claims, wherein R3 and R4 are each hydrogen.

11. Asymmetric imidazolium salt of formula 1D obtained by the method according to one of the preceding claims: 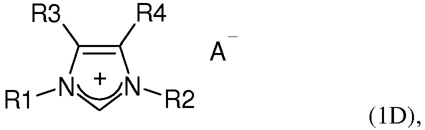 wherein
R1 is an aromatic group selected from the group consisting of 2,4,6-trimethylphenyl, 3,5-dinitrophenyl, 2,4,6-tris(trifluoromethyl)phenyl, 2,4,6-trichlorophenyl, and hexafluorophenyl,
R2 is a secondary cyclic aliphatic alkyl group,
R3 and R4 are selected independently of one another from the group consisting of hydrogen, a halide and an alkyl group, and
A⁻ is an anion selected from the group consisting of a tetrafluoroborate anion, a hexafluorophosphate anion, an acetate anion, a hexafluoroantimony anion, a tetrakis[(3,5-trifluoromethyl)phenyl]borate anion and a halide anion.

12. Asymmetric imidazolium salt according to Claim 11, wherein R2 is selected from the group consisting of cyclopentyl, cyclohexyl, cycloheptyl, cyclooctyl, cyclodecyl, cyclododecyl, and cyclopentadecyl.

13. Asymmetric imidazolium salt according to one of Claims 11 and 12, wherein R3 and R4 are each hydrogen.
